# EUROPEAN PATENT SPECIFICATION

(11) **EP 3 520 729 B1**
(45) Date of publication and mention of the grant of the patent: **17.01.2024**
(21) Application number: 17854493.8
(22) Date of filing: 30.06.2017
(51) Int. Cl.: A61B 34/30, A61B 34/37, B25J 18/00, B25J 9/08, B25J 11/00, A61B 17/00

(54) **SURGICAL ROBOT SYSTEM**
CHIRURGISCHES ROBOTERSYSTEM
SYSTÈME DE ROBOT CHIRURGICAL

(30) Priority: 28.09.2016 CN 201610860701
(43) Date of publication of application: 07.08.2019
(73) Proprietor: Shanghai Microport Medbot (Group) Co., Ltd., Shanghai 201203 (CN)
(72) Inventor: HE, Chao, Shanghai 201203 (CN); YUAN, Shuai, Shanghai 201203 (CN); WANG, Changchun, Shanghai 201203 (CN); LI, Tao, Shanghai 201203 (CN); HE, Yuyuan, Shanghai 201203 (CN)
(74) Representative: Patentship Patentanwaltsgesellschaft mbH
(86) International application number: PCT/CN2017/091120
(87) International publication number: WO 2018/059039

(56) References cited:
- WO-A1-2014/012163
- WO-A1-2015/142955
- CN-A- 102 018 574
- CN-A- 102 764 157
- CN-A- 102 973 317
- CN-A- 102 973 317
- CN-A- 103 027 754
- CN-A- 105 726 126
- CN-A- 105 726 126
- CN-A- 106 236 276
- CN-U- 202 143 653

## Description

### TECHNICAL FIELD

The present invention relates to the technical field of medical instruments, and in particular, to a surgical robotic system.

### BACKGROUND OF THE INVENTION

The minimally invasive surgery refers to a new technique of carrying out surgery in human body through laparoscope, thoracoscope and other endoscopes, and has the advantages of minimal wound, slight pain and less bleeding, which can effectively reduce the recovery time and inadaptability of patients and avoid some harmful side effects of traditional surgery therefore. Early minimally invasive surgery has the defects that the degree of freedom of surgical tools for doctor is relatively reduced due to the limitation of pores at body surface, and the operation direction may also be opposite to the desired direction, which increases the difficulty for the doctor to carry out surgery. As a result, the doctor can carry out the surgery smoothly only after a long-term relevant training.

With the development of science and technology, especially the development of robotic technology, a better solution has been found to the problems in the early minimally invasive surgery, and thus the minimally invasive surgical robotic system has been researched and developed. The minimally invasive surgical robotic system enables the doctor to observe the tissue characteristics of the patient's body through a two-dimensional or three-dimensional display device at the surgeon's control console, and remotely manipulate manipulators and surgical instruments on the patient side cart to operate the surgery. It make that the doctor operate the minimally invasive surgery with the feeling as the traditional open surgery. Meanwhile, it reduces the difficulty of the doctor in the operation, improves the efficiency and safety of the surgery, and makes a breakthrough in the realization of remote surgery. In view of the superiority of the surgical robotic system, countries in the world are actively doing research in related fields and have produced some products and prototypes.

The researches of minimally invasive surgical robotic devices and/or systems enable doctors not only operate with minimal wound and the same viewing angle and operational experience as traditional opening surgery but also operate away from a patient's location, or next to the patient in the ward, or under the remotely control.

In a remote surgery, the surgeon uses some form of remote controller, such as a servo mechanism, to manipulate the movement of the surgical instrument rather than directly holding and moving the instrument. In a remote surgical system, the surgeon manipulates a master manipulator of the surgeon's control console to control the movement of the surgical instrument of the servo mechanism and then operate on the patient. Further, to achieve this type of surgical procedure, there must be a support manipulator in the system/device to support the surgical instrument and drive the surgical instrument to move. Moreover, the spatial arrangement of the surgical instruments severely affects the convenience and mobility of the surgical instrument's adjustment, the compactness of the patient side cart, and the collision avoidance between the support manipulator of the surgical instrument in the working space. Similar minimally invasive robotic device/system stents have been proposed in US Patent No. 6,246,200 B1 and No. 6,788,018 B1. Although the solution proposed in the prior art has been proved to be efficient, beneficial and convenient, there are still some problems of motion interference or difficulty in reaching some desired position. Therefore, further improvements are still needed, to improve the safety, convenience, comfort and functionality of the manipulation of the minimally invasive surgical robotic system. A surgical robotic system is also disclosed in document WO2015/142955A1.

### SUMMARY OF THE INVENTION

It is an object of the present invention to provide a surgical robotic system, which enables the manipulator to be adjusted and positioned more conveniently and to have a larger working space during the movement, avoids or inhibits collision between different manipulators more effectively, and is also more suitable for the demands of different surgical scenarios, so that the safety, convenience, comfort or functionality of the manipulation of the minimally invasive surgical robotic system can be improved.

Based on the foregoing object, the present invention provides a surgical robotic system according to claim 1.

In the surgical robotic system provided by the present application, the module is simple in configuration and small in size. Through the optimized module and optimized manipulator, the adjustment of the manipulator is able to be completed more conveniently, the motion space of the manipulator is able to be enlarged, and the possibility of collisions during the motions of manipulators is able to be reduced and/or suppressed, thereby improving the safety, convenience, comfort or functionality of the minimally invasive surgical robotic system.

### BRIEF DESCRIPTION OF THE DRAWINGS

FIG. 1 is a schematic structural diagram of a surgical robotic system according to Embodiment I of the present invention;
FIG. 2 is a schematic structural diagram of a mounting base and a support component according to Embodiment I of the present invention;
FIG. 3 is a schematic structural diagram of a orienting platform according to Embodiment I of the present invention;
FIG. 4 is a schematic structural diagram of a manipulator according to Embodiment I of the present invention;
FIG. 5 is a schematic structural diagram of another orienting platform according to Embodiment I of the present invention;
FIG. 6 is a schematic structural diagram of a mounting base and a support component according to Embodiment II of the present invention;
FIG. 7 is a schematic structural diagram of a mounting base and a support component according to Embodiment III of the present invention;
FIG. 8 is a schematic structural diagram of a orienting platform according to Embodiment IV of the present invention;
FIG. 9 is a schematic structural diagram of another orienting platform according to Embodiment IV of the present invention;
FIG. 10 is a schematic structural diagram of a orienting platform and a manipulator according to Embodiment V of the present invention;
FIG. 11 is a schematic structural diagram of another orienting platform and manipulator according to Embodiment V of the present invention;
FIG. 12 is a schematic structural diagram of a orienting platform according to Embodiment VI of the present invention;
FIG. 13 is a schematic structural diagram of a orienting platform according to Embodiment VI of the present invention;
FIG. 14 is a schematic structural diagram of a orienting platform according to Embodiment VII of the present invention; and
FIG. 15 is a schematic structural diagram of a orienting platform according to Embodiment VII of the present invention.

### DETAILED DESCRIPTION OF THE INVENTION

The surgical robotic system proposed by the present invention will be further described in detail below with reference to the accompanying drawings and specific embodiments. Advantages and features of the present invention will be apparent from the description and appended claims below. It should be noted that the drawings are in a very simplified form and not necessarily presented to scale, for the only purpose to facilitate convenient and explicit description of embodiments of the present invention. In particular, the drawings are shown for different emphases, and often adopted different proportions.

The core idea of the present application is to provide a surgical robotic system comprising a module and a manipulator. The module comprises a mounting base, a support component connected to the mounting base, and a orienting platform connected to the support component. The manipulator is connected to the orienting platform in the module including at least two degrees of freedom. Thus, the manipulator is able to be adapted to the surgical operations at different position, thereby improving the safety, convenience, comfort or functionality of the manipulation of the minimally invasive surgical robotic system.

Specifically, please refer to the following examples of surgical robotic systems. In the following examples of surgical robotic systems, the adjustable support component of the module, the adjustable orienting platform of the module, and the adjustable manipulator each enables to achieve the foregoing object, thereby improving the safety, convenience, comfort or functionality of the manipulation of the minimally invasive surgical robotic system.

### [Embodiment I]

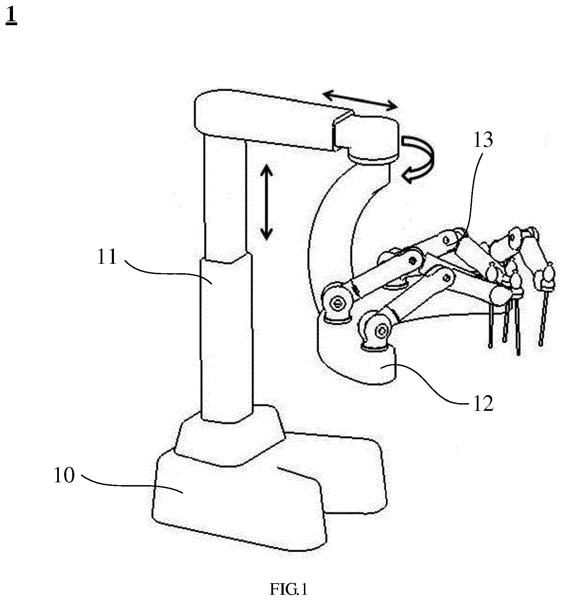

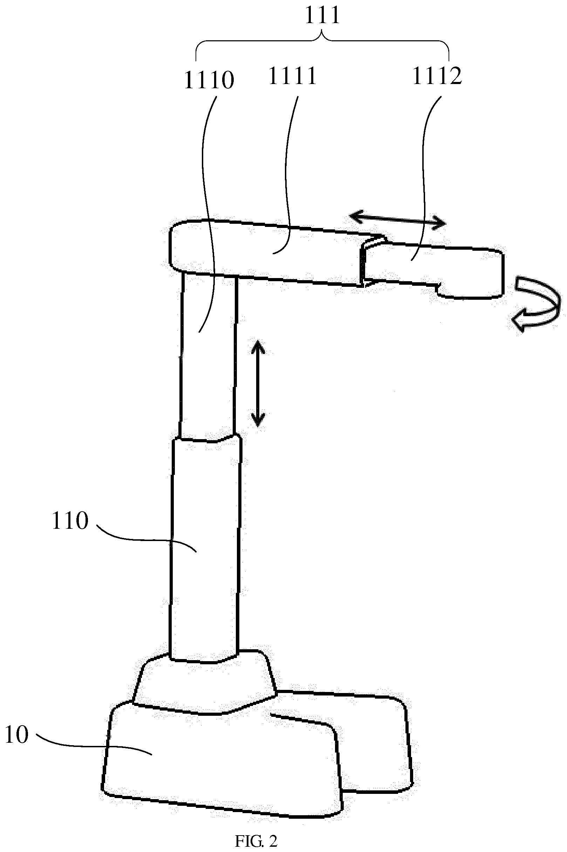

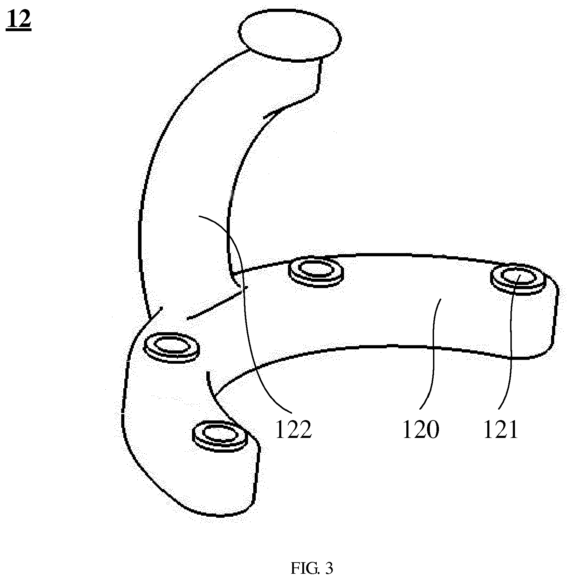

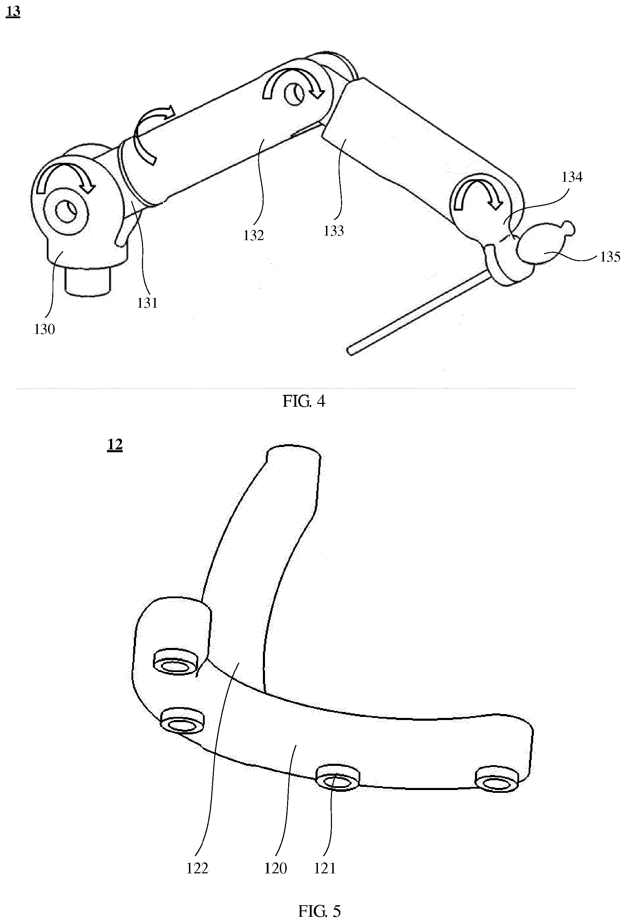

Referring to FIGs. 1-4, where FIG. 1 is a schematic structural diagram of a surgical robotic system according to Embodiment I of the present invention; FIG. 2 is a schematic structural diagram of a mounting base and a support component according to Embodiment I of the present invention; FIG. 3 is a schematic structural diagram of a orienting platform according to Embodiment I of the present invention; and FIG. 4 is a schematic structural diagram of a manipulator according to Embodiment I of the present invention.

As shown in FIGs. 1-4, in Embodiment I of the present application, the surgical robotic system 1 comprises a module and a manipulator 13. The module comprises a mounting base 10, a support component 11 connected to the mounting base 10, and a orienting platform 12 connected to the support component 11, wherein the orienting platform 12 is connected to the manipulator 13.

Specifically, the support component 11 comprises a first support part 110 secured to the mounting base 10 and a second support part 111 connected to the first support part 110, wherein the second support part 111 is movable along an extending direction of the first support part 110, that is, the support component 11 is variable in terms of height, having one degree of freedom. Here, the second support part 111 is able to move along the extending direction of the first support part 110, so that the relative height between the manipulator 13 and the mounting base 10 is able to be coarsely adjusted.

Still referring to FIGs. 1 and 2, further, the second support part 111 comprises a first support branch 1110 connected to the first support part 110, a second support branch 1111 connected to the first support branch 1110, and a third support branch 1112 connected to the second support branch 1111, wherein an extending direction of the first support branch 1110 is perpendicular to that of the third support branch 1112. Further, the first support branch 1110 is able to move along the extending direction of the first support part 110, an included angle between the second support branch 1111 and the first support branch 1110 is 90°, and the third support branch 1112 is able to move along the extending direction of the second support branch 1111, that is, the second support part 111 is variable in terms of the length, having one degree of freedom. Thus, the relative position between the manipulator 13 and the mounting base 10, along the extending direction of the second support branch 1111, is able to be coarsely adjusted.

Still referring to FIGs. 1 and 3, in the embodiment of the present application, the orienting platform 12 is rotatably connected to the support component 11, forming one degree of freedom. Specifically, the orienting platform 12 comprises a top tray part120, and at least one mounting part 121 configured to connect the manipulator 13 is distributed on the top tray part120. In the embodiment of the present application, there is a plurality of mounting parts 121, and the plurality of mounting part 121 are distributed on an upper surface of the top tray part120. In other embodiments of the present application, the plurality of mounting parts 121 may also be distributed on a lower surface of the top tray part120, referring to FIG. 5 for details. A plurality of manipulators 13 can be simultaneously mounted through the plurality of mounting parts 121, and the manipulators 13 are rotatably connected to the top tray part120, thereby increasing the working space of the manipulator 13 at a horizontal plane.

The connection modes between the mounting parts 121 and the top tray part120 and the connection modes between the manipulator 13 and the mounting part 121 can be various, and the embodiment of the present application is not limited thereto. For example, the mounting part 121 is rotatably connected to the top tray part120, and the manipulator 13 is fixedly connected to the mounting part 121, so that the rotatable connection between the manipulator 13 and the top tray part120 is able to be established. For another example, the mounting part 121 is fixedly connected to the top tray part120, and the manipulator 13 is rotatably connected to the mounting part 121, so that the rotatable connection between the manipulator 13 and the top tray part120 is established; or the mounting part 121 is rotatably connected to the top tray part120, and the manipulator 13 is also rotatably connected to the mounting part 121, so that the rotatable connection between the manipulator 13 and the top tray part120 is also established.

Further, the orienting platform 12 further comprises a suspension arm 122, the top tray part120 is fixed on the suspension arm 122, and the suspension arm 122 is rotatably connected to the support component 11. Preferably, both the suspension arm 122 and the top tray part120 are both arc-shaped for the arc-shaped structure enabling to make the structure of the surgical robotic system 1 more compact, and at the same time, provide a greater operating space to the manipulator 13.

Still referring to FIGs. 1 and 4, in the present application, the manipulator 13 is an active remote-center-of-motion manipulator, comprising a robotic arm and a surgical instrument connected to the robotic arm. The active remote-center-of-motion manipulator refers to that, except for the degree of freedom of the surgical instrument, the robotic arm supporting the surgical instrument has a spatial configuration of at least four degrees of freedom, thereby enabling the surgical instrument to move around a certain point in the space through algorithm control; and the point is the active remote-center-of-motion point. In the embodiment of the present application, the manipulator 13 specifically comprises a shoulder part 130, an arm support part 131 connected to the shoulder part 130, an upper arm part 132 connected to the arm support part 131, a lower arm part 133 connected to the upper arm part 132, an instrument support part 134 connected to the lower arm part 133, and a surgical instrument 135 connected to the instrument support part 134.

Preferably, the arm support part 131 is swingable relative to the shoulder part 130, and the upper arm part 132 is axially rotatable relative to the arm support part 131. The lower arm structure 133 is swingable relative to the upper arm part 132, and the instrument support part 134 is swingable relative to the lower arm part 133. More preferably, the surgical instrument 135 is able to perform a rotational motion. Thus, the manipulator 13 has four or more degrees of freedom, thereby providing a configuration basis for the active remote-center-of-motion manipulator.

Further, the mounting base 10 may be provided with a caster part, so that the surgical robotic system 1 is able to move freely; or the mounting base 10 is mounted with a motion control part, so that the surgical robotic system 1 is able to move under controlled conditions; or the mounting base 10 is mounted with a horizontal fixable support part, so that the surgical robotic system 1 is able to horizontally fix and support the foregoing part.

In summary, in the surgical robotic system 1 provided in Embodiment I of the present application, the module includes at least three degrees of freedom, which is able to provide the manipulator 13 with coarse adjustments in multiple directions, conveniently complete the adjustment of the manipulator and expand the motion space of the manipulator, thereby improving the safety, convenience, comfort or functionality of the minimally invasive surgical robotic system.

### [Embodiment II]

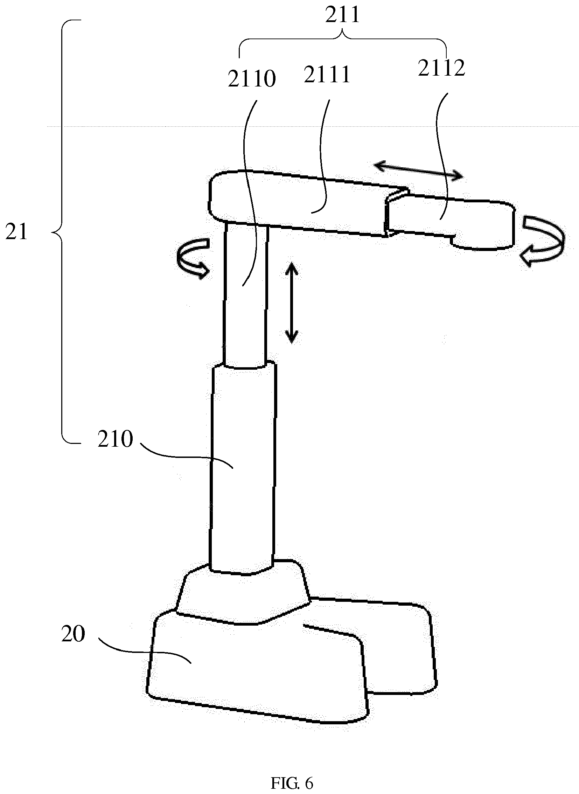

Referring to FIG. 6, a schematic structural diagram of a mounting base and a support component according to Embodiment II of the present application. As shown in FIG. 6, in the embodiment of the present application, the support component 21 comprises a first support part 210 secured to the mounting base 20 and a second support part 211 connected to the first support part 210, wherein the second support part 211 is movable along the extending direction of the first support part 210. Further, the second support part 211 comprises a first support branch 2110 connected to the first support part 210, a second support branch 2111 connected to the first support branch 2110, and a third support branch 2112 connected to the second support branch 2111, wherein the extending direction of the first support branch 2110 is perpendicular to that of the third support branch 2112. Further, the first support branch 2110 is movable along the extending direction of the first support part 210. Preferably, an included angle between the second support branch 2111 and the first support branch 2110 is 90°, and the third support branch 2112 is movable along the extending direction of the second support branch 2111.

In the embodiment of the present application, apart from moving along the extending direction of the first support part 210, the second support part 211 is also able to move relative to the first support part 210. Further, the first support branch 2110 is able to be rotatably connected to the first support part 210. That is, Embodiment II of the present application differs from Embodiment I in that the second support part 211 is further rotatably connected to the first support part 210. Thus, the module is added with one degree of freedom and has at least four degrees of freedom, which improves the convenience of adjustment of the manipulator and expands the motion space of the manipulator.

Other parts that are not described in Embodiment II may correspondingly refer to Embodiment I or other embodiments, and would not be described in Embodiment II again.

### [Embodiment III]

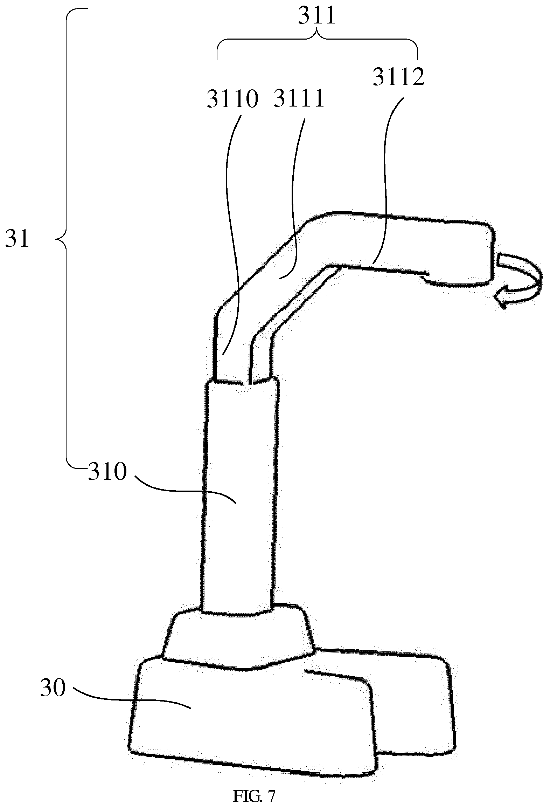

Referring to FIG. 7, a schematic structural diagram of a mounting base and a support component according to Embodiment III of the present application. As shown in FIG. 7, in the embodiment of the present application, the support component 31 comprises a first support part 310 secured to the mounting base 30 and a second support part 311 connected to the first support part 310. Further, the second support part 311 comprises a first support branch 3110 connected to the first support part 310, a second support branch 3111 fixedly connected to the first support branch 3110, and a third support branch 3112 fixedly connected to the second support branch 3111, wherein the extending direction of the first support branch 3110 is perpendicular to that of the third support branch 3112. Preferably, an included angle between the second support branch 3111 and the first support branch 3110 is greater than 90° and less than 180°, and an included angle between the third support branch 3112 and the second support branch 3111 is greater than 90° and less than 180°. That is, through the foregoing arrangement, the first support branch 3110, the second support branch 3111, and the third support branch 3112 form an arc-like configuration, so that the configuration of the surgical robotic system is more compact. In this embodiment, the module has only two degrees of freedom.

Other parts that are not described in Embodiment III may correspondingly refer to other embodiments, and would not be described in Embodiment III again.

### [Embodiment IV]

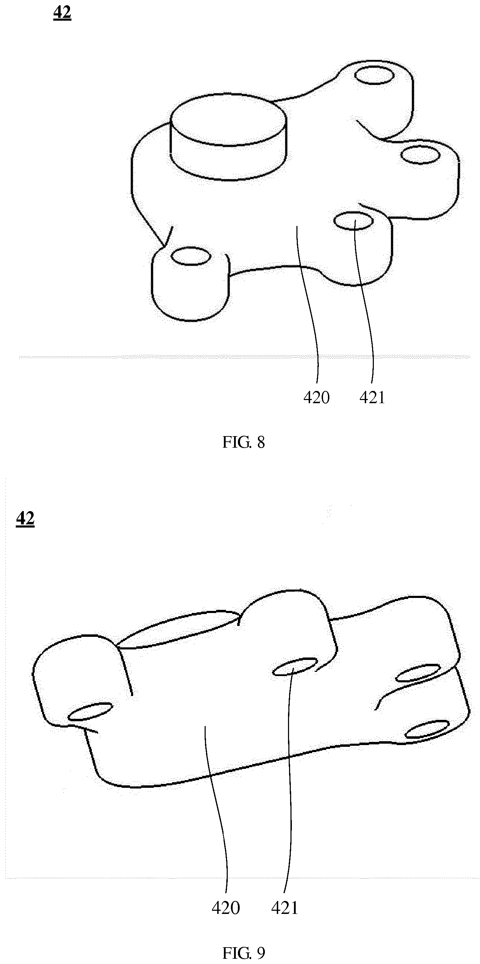

Referring to FIG. 8, a schematic structural diagram of a orienting platform according to Embodiment IV of the present application. As shown in FIG. 8, in the embodiment of the present application, the orienting platform 42 comprises a top tray part 420, and a plurality of mounting parts 421 are distributed on the top tray part 420. In the embodiment of the present application, the orienting platform 42 is claw-shaped. That is, the top tray part 420 includes a palm and fingers, the palm is rotatably connected to the support component, and the mounting parts 421 are disposed on upper surfaces of the fingers. The orienting platform 42 is connected to the manipulator through the mounting part 421. Further, the mounting part 421 is disposed on the upper surface of one of the fingers. In other embodiments of the present application, the mounting part 421 may also be disposed on the lower surfaces of the fingers, that is, the mounting part 421 are disposed on the lower surface of the top tray part 420, referring to FIG. 9 for details. A plurality of manipulators can be simultaneously mounted by providing a plurality of mounting parts 421, thereby facilitating to perform complicated surgical operations.

Other parts that are not described in Embodiment IV may correspondingly refer to other embodiments, and would not be described in Embodiment IV again.

### [Embodiment V]

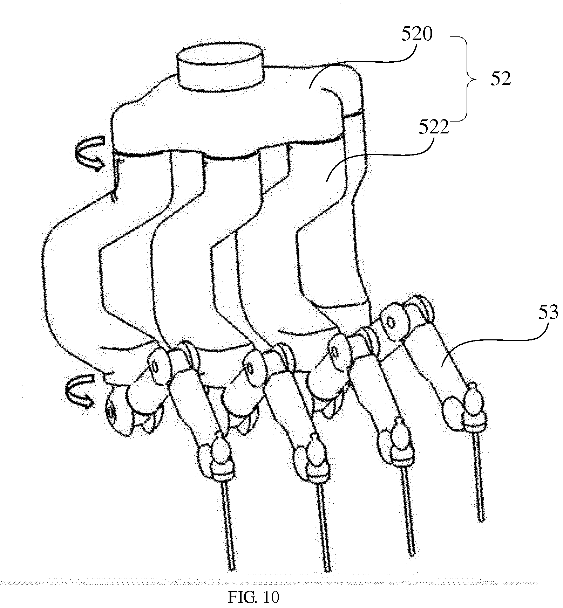

Referring to FIG. 10, a schematic structural diagram of a orienting platform and a manipulator according to Embodiment V of the present application. As shown in FIG. 10, the orienting platform 52 comprises a top tray part 520, and at least one mounting part (not shown in FIG. 10) is distributed on the top tray part 520. In the embodiment of the present application, the orienting platform 52 further comprises a plurality of base extensions 522, and each base extension 522 is connected to one mounting part. Here, the base extension 522 is able to rotate relative to the top tray part 520. The relative rotation between the base extension 522 and the top tray part 520 is able to be established through the rotational connection between the base extension 522 and the mounting part, or through the rotational connection between the mounting part and the top tray part 520. Preferably, the base extension 522 is of an arc-like configuration.

Further, the manipulator 53 is connected to the mounting part through the base extension 522. Specifically, one manipulator 53 is connected (preferred a rotational connection) to one base extension 522, and the base extension 522 (connected to one manipulator 53) is connected to the mounting part.

In the embodiment of the present application, a first rotational connection was formed between the base extension 522 and the top tray part520, and a second rotational connection was formed the manipulator 53 and the base extension 522. The axis of the first rotational connection is parallel to the axis of the second rotational connection. Preferably, the axis of the first rotational connection is co-linear with the axis of the second rotational connection.

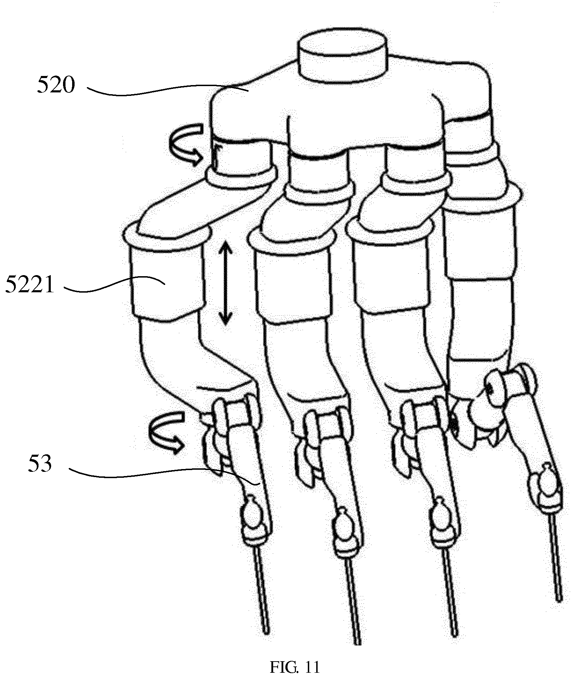

Furthermore, as shown in FIG. 11, the base extension 522 comprises a telescopic moving joint 5221, wherein the telescopic moving joint 5221 is able to elongate or shorten along the axial direction of the base extension 522 to change the axial length of the base extension 522. The telescopic moving joint 5221 is able to drive the manipulator 53 (here is an active remote-center-of-motion manipulator) to move away from and close to the top tray part 520.

Preferably (referring to FIG. 1 correspondingly), the rotary shaft of the orienting platform 52 rotating relative to the support component is parallel to the rotary shaft of each base extension 522 rotating relative to the top tray part520. And, the distance between the two rotary shafts is able to be designed according to the needs of the surgical robotic system.

Other parts that are not described in Embodiment V may correspondingly refer to other embodiments, and would not be described in Embodiment V again.

### [Embodiment VI]

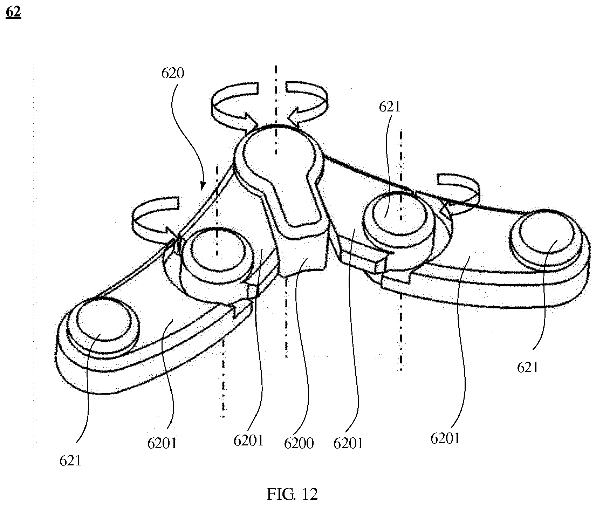

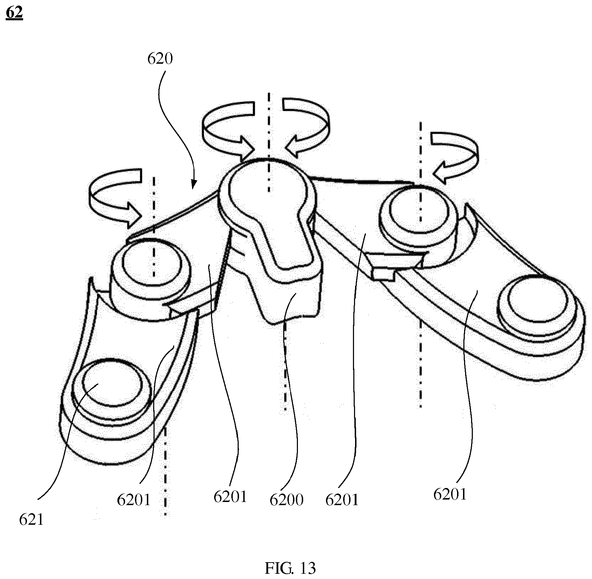

Referring to FIGs. 12 and 13, FIG. 12 is a schematic structural diagram of a orienting platform according to Embodiment VI of the present application, and FIG. 13 is a schematic structural diagram of a orienting platform according to Embodiment VI of the present application. As shown in FIGs. 12 and 13, in the embodiment of the present application, the orienting platform 62 comprises a top tray part 620, and at least one mounting part 621 is distributed on the top tray part 620. The top tray part 620 is able to be connected to a plurality of manipulators through the plurality of mounting part 621, thereby facilitating to perform complicated surgical operations. Further, the top tray part 620 comprises a central support 6200 and a plurality of rotating members 6201, one mounting part 621 is distributed on each rotating member 6201, and each rotating member 6201 is able to rotate relative to the adjacent central support 6200 or another rotating member 6201. Further, when the two rotating members 6201 are adjacent, the rotating member away from the center support 6200 is rotatably connected to the rotating member 6201 close to the support bracket 6200 through the mounting part 621 disposed on the rotating member close to the center support 6200. The mounting part 621 is driven to rotate through the rotation of the rotating member 6201 and then the position relationship between the manipulators is driven to change, so that the manipulator is adjusted more flexibly. Preferably, a brake element is mounted at the rotating joint of each rotating member 6201, thereby ensuring that each rotating member 6201 is able to maintain a constant position and a high joint stiffness after moving to a certain position.

Other parts that are not described in Embodiment VI may correspondingly refer to other embodiments, and would not be described in Embodiment VI again.

### [Embodiment VII]

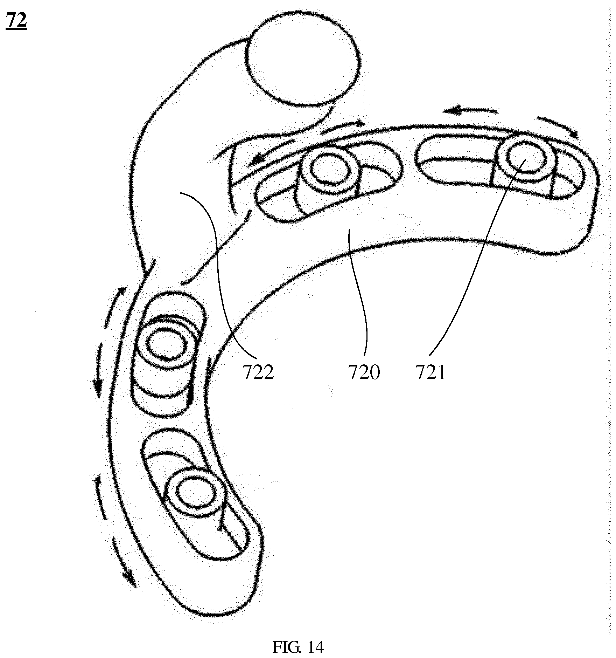

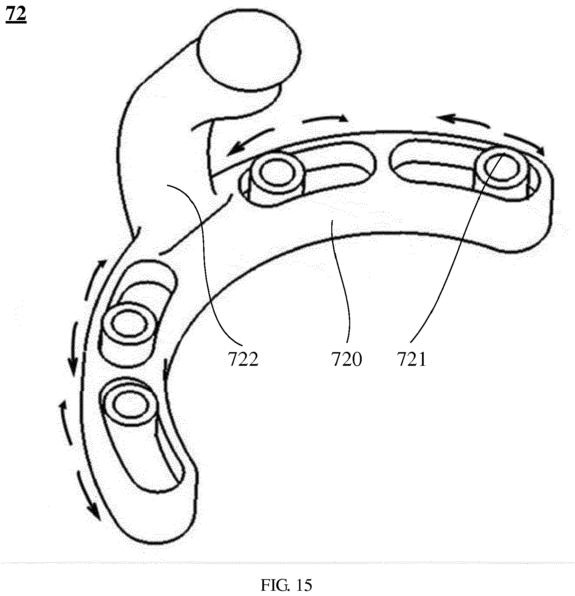

Referring to FIGs. 14 and 15, FIG. 14 is a schematic structural diagram of a orienting platform according to Embodiment VII of the present application, and FIG. 15 is a schematic structural diagram of a orienting platform according to Embodiment VII of the present application. As shown in FIGs. 14 and 15, in the embodiment of the present application, the orienting platform 72 comprises a top tray part720, and at least one mounting part 721 is distributed on the top tray part 720. A plurality of manipulators is able to be simultaneously mounted on the top tray part720 through the mounting part 721, thereby facilitating to perform complicated surgical operations. Further, the position of each mounting part 721 on the top tray part720 is able to be changed. In the embodiment of the present application, a plurality of moving slots are formed on the top tray part720, each mounting part 721 is located into a moving slot, and each mounting part 721 changes the position by moving within the moving slot. Further, the rotational connections between the manipulators and the top tray part 720 are established by the mounting parts 721 located in the moving slots. Thus, the changes of the relative position and gesture between the manipulators are able to be obtained, which makes the adjustment of the manipulator more convenient. Further, the orienting platform 72 further comprises a suspension arm 722, the top tray part720 is fixed on the suspension arm 722, and the suspension arm 722 is rotatable relative to the support component. In the embodiment of the present application, both the suspension arm 722 and the top tray part 720 are arc-shaped, and the arc-shaped configuration enables to provide a surgical robotic system with a more compact configuration, a greater operating space for the manipulator, and a more flexible adjustment of the manipulators.

Other parts that are not described in Embodiment VII may correspondingly refer to other embodiments, and would not be described in Embodiment VII again.

In view of the above, in the surgical robotic system provided in the embodiments of the present application, the module is simple in configuration and small in size. Through the optimized module and manipulator, the adjustment of the manipulator is able to be completed more conveniently, the motion space of the manipulator is able to be enlarged, and the possibility of collisions during the motions of manipulators is able to be reduced and/or suppressed, thereby improving the safety, convenience, comfort or functionality of the minimally invasive surgical robotic system.

## Claims

1. A surgical robotic system (1), comprising a module and a manipulator (13), wherein the module comprises a mounting base (10, 20, 30), a support component (11, 21, 31) connected to the mounting base (10, 20, 30), and a orienting platform (12, 42, 52, 62, 72) connected to the support component (11, 21, 31); wherein the manipulator (13) is connected to the orienting platform (12, 42, 52, 62, 72) of the module; the manipulator (13) comprises a robotic arm and a surgical instrument (135) connected to the robotic arm, and the manipulator (13) is an active remote-center-of-motion manipulator (13), wherein the robotic arm supporting the surgical instrument has a spatial configuration of at least four degrees of freedom, enabling the surgical instrument to move around a certain point in the space through algorithm control, wherein the orienting platform (12, 42, 52, 62, 72) comprises a top tray part (120, 420, 520, 620, 720) and a suspension arm (122), wherein at least one mounting part (121, 421, 621, 721) is distributed on the top tray part (120, 420, 520, 620, 720) to directly or indirectly connect the manipulator (13), and **characterised in that** the top tray part (120, 420, 520, 620, 720) is secured to the suspension arm (122, 722), and the suspension arm (122, 722) is rotatably connected to the support component (11, 21, 31).

2. The surgical robotic system (1) according to claim 1, wherein the support component (11, 21, 31) comprises a first support part (110, 210, 310) secured to the mounting base (10, 20, 30) and a second support part (111, 211, 311) connected to the first support part (110, 210, 310).

3. The surgical robotic system (1) according to claim 2, wherein the second support part (111, 211, 311) is movable along an extending direction of the first support part (110, 210, 310) and/or the second support part (111, 211, 311) is rotatable relative to the first support part (110, 210, 310).

4. The surgical robotic system (1) according to claim 2, wherein the second support part (111, 211, 311) comprises a first support branch (1110, 2110, 3110) connected to the first support part (110, 210, 310), a second support branch (1111, 2111, 3111) connected to the first support branch (1110, 2110, 3110), and a third support branch (1112, 2112, 3112) connected to the second support branch (1111, 2111, 3111), wherein an extending direction of the first support branch (1110, 2110, 3110) is perpendicular to an extending direction of the third support branch (1112, 2112, 3112), preferably,
the third support branch (1112, 2112, 3112) is movable along an extending direction of the second support branch (1111, 2111, 3111), and/or the first support branch (1110, 2110, 3110) is movable along the extending direction of the first support part (110, 210, 310).

5. The surgical robotic system (1) according to claim 1, wherein the orienting platform (12, 42, 52, 62, 72) is rotatable relative to the support component (11, 21, 31).

6. The surgical robotic system (1) according to claim 1, wherein the top tray part (120, 420, 520, 620, 720) is arc-shaped or claw-shaped.

7. The surgical robotic system (1) according to claim 6, wherein each mounting part (121, 421, 621, 721) is rotatable relative to the top tray part (120, 420, 520, 620, 720).

8. The surgical robotic system (1) according to claim 6, wherein each mounting part (121, 421, 621, 721) is movable on the top tray part (120, 420, 520, 620, 720).

9. The surgical robotic system (1) according to claim 1, wherein the top tray part (120, 420, 520, 620, 720) comprises a central support (6200) and a plurality of rotating members (6201), one mounting part (121, 421, 621, 721) is distributed on each rotating member (6201), the central support is rotatably connected to an adjacent rotating member (6201), and each rotating member (6201) is rotatably connected to an adjacent rotating member (6201) through the corresponding mounting part (121, 421, 621, 721).

10. The surgical robotic system (1) according to claim 1, wherein the orienting platform (12, 42, 52, 62, 72) further comprises a plurality of base extensions (522), each base extension (522) forms a first rotating connection with the top tray part (120, 420, 520, 620, 720), each base extension (522) forms a second rotating connection with the manipulator (13), and an axis of the first rotating connection is parallel to an axis of the second rotating connection, preferably,
an axis of the first rotating connection is co-linear with an axis of the second rotating connection.

11. The surgical robotic system (1) according to claim 10, wherein the base extension (522) comprises a telescopic moving joint (5221) configured to change a length of the base extension (522).

12. The surgical robotic system (1) according to claim 1, wherein the suspension arm (122, 722) and/or the top tray part (120, 420, 520, 620, 720) is arc-shaped.

13. The surgical robotic system (1) according to claim 1, wherein the robotic arm comprises a shoulder part (130), an arm support part (131) connected to the shoulder part (130), an upper arm part (132) connected to the arm support part (131), a lower arm part (133) connected to the upper arm part (132), and an instrument support part (134) connected to the lower arm part (133); wherein the instrument support part (134) is configured to connect with the surgical instrument (135).

14. The surgical robotic system (1) according to claim 13, wherein the arm support part (131) is pivotably connected to the shoulder part (130), the upper arm part (132) is rotatably connected to the arm support part (131), the lower arm part (133) is pivotably connected to the upper arm part (132), and the instrument support part (134) is pivotably connected to the lower arm part (133).

15. The surgical robotic system (1) according to claim 1, wherein the mounting base (10, 20, 30) is mounted with a caster part, a fixed support part and/or a motion control part.

## Patentansprüche

1. Chirurgisches Robotersystem (1), umfassend ein Modul und einen Manipulator (13), wobei das Modul eine Montagebasis (10, 20, 30), eine mit der Montagebasis (10, 20, 30) verbundene Trägerkomponente (11, 21, 31), und eine mit der Trägerkomponente (11, 21, 31) verbundene Orientierungsplattform (12, 42, 52, 62, 72) umfasst; wobei der Manipulator (13) mit der Orientierungsplattform (12, 42, 52, 62, 72) des Moduls verbunden ist; wobei der Manipulator (13) einen Roboterarm und ein mit dem Roboterarm verbundenes chirurgisches Instrument (135) umfasst, und wobei der Manipulator (13) ein aktiver Manipulator (13) mit entferntem Bewegungszentrum ist, wobei der das chirurgische Instrument tragende Roboterarm eine räumliche Konfiguration mit mindestens vier Freiheitsgraden aufweist, die es dem chirurgischen Instrument ermöglicht, sich durch algorithmische Steuerung um einen bestimmten Punkt im Raum zu bewegen, wobei die Orientierungsplattform (12, 42, 52, 62, 72) einen oberen Einsatzteil (120, 420, 520, 620, 720) und einen Aufhängungsarm (122) umfasst, wobei mindestens ein Montageteil (121, 421, 621, 721) auf dem oberen Einsatzteil (120, 420, 520, 620, 720) zur direkten oder indirekten Anbindung des Manipulators (13) verteilt ist, und **dadurch gekennzeichnet, dass** der obere Einsatzteil (120, 420, 520, 620, 720) an dem Aufhängungsarm (122, 722) befestigt ist, und wobei der Aufhängungsarm (122, 722) mit der Trägerkomponente (11, 21, 31) rotierbar verbunden ist.

2. Chirurgisches Robotersystem (1) nach Anspruch 1, wobei die Trägerkomponente (11, 21, 31) einen an der Montagebasis (10, 20, 30) befestigten ersten Trägerteil (110, 210, 310) und einen mit dem ersten Trägerteil (110, 210, 310) verbundenen zweiten Trägerteil (111, 211, 311) umfasst.

3. Chirurgisches Robotersystem (1) nach Anspruch 2, wobei der zweite Trägerteil (111, 211, 311) entlang einer Erstreckungsrichtung des ersten Trägerteils (110, 210, 310) bewegbar ist, und/oder wobei der zweite Trägerteil (111, 211, 311) relativ zu dem ersten Trägerteil (110, 210, 310) rotierbar ist.

4. Chirurgische Robotersystem (1) nach Anspruch 2, wobei der zweite Trägerteil (111, 211, 311) einen mit dem ersten Trägerteil (110, 210, 310) verbundenen ersten Trägerzweig (1110, 2110, 3110), einen mit dem ersten Trägerzweig (1110, 2110, 3110) verbundenen zweiten Trägerzweig (1111, 2111, 3111), und einen mit dem zweiten Trägerzweig (1111, 2111, 3111) verbundenen dritten Trägerzweig (1112, 2112, 3112) umfasst, wobei eine Erstreckungsrichtung des ersten Trägerzweigs (1110, 2110, 3110) senkrecht zu einer Erstreckungsrichtung des dritten Trägerzweigs (1112, 2112, 3112) ist, vorzugsweise,
wobei der dritte Trägerzweig (1112, 2112, 3112) entlang einer Erstreckungsrichtung des zweiten Trägerzweigs (1111, 2111, 3111) bewegbar ist, und/oder wobei der erste Trägerzweig (1110, 2110, 3110) entlang der Erstreckungsrichtung des ersten Trägerteils (110, 210, 310) bewegbar ist.

5. Chirurgisches Robotersystem (1) nach Anspruch 1, wobei die Orientierungsplattform (12, 42, 52, 62, 72) relativ zu der Trägerkomponente (11, 21, 31) rotierbar ist.

6. Chirurgisches Robotersystem (1) nach Anspruch 1, wobei der obere Einsatzteil (120, 420, 520, 620, 720) bogenförmig oder klauenförmig ist.

7. Chirurgisches Robotersystem (1) nach Anspruch 6, wobei jeder Montageteil (121, 421, 621, 721) relativ zu dem oberen Einsatzteil (120, 420, 520, 620, 720) rotierbar ist.

8. Chirurgisches Robotersystem (1) nach Anspruch 6, wobei jeder Montageteil (121, 421, 621, 721) auf dem oberen Einsatzteil (120, 420, 520, 620, 720) bewegbar ist.

9. Chirurgisches Robotersystem (1) nach Anspruch 1, wobei der obere Einsatzteil (120, 420, 520, 620, 720) einen zentralen Träger (6200) und eine Vielzahl von Rotationselementen (6201) umfasst, wobei ein Montageteil (121, 421, 621, 721) auf jedem Rotationselement (6201) verteilt ist, wobei der zentrale Träger mit einem benachbarten Rotationselement (6201) rotierbar verbunden ist, und wobei jedes Rotationselement (6201) mit einem benachbarten Rotationselement (6201) durch den entsprechenden Montageteil (121, 421, 621, 721) rotierbar verbunden ist.

10. Chirurgisches Robotersystem (1) nach Anspruch 1, wobei die Orientierungsplattform (12, 42, 52, 62, 72) ferner eine Vielzahl von Basisverlängerungen (522) umfasst, wobei jede Basisverlängerung (522) eine erste Rotationverbindung mit dem oberen Einsatzteil (120, 420, 520, 620, 720) bildet, wobei jede Basisverlängerung (522) eine zweite Rotationsverbindung mit dem Manipulator (13) bildet, und wobei eine Achse der ersten Rotationsverbindung parallel zu einer Achse der zweiten Rotationsverbindung ist, vorzugsweise,
wobei eine Achse der ersten Rotationsverbindung kolinear mit einer Achse der zweiten Rotationsverbindung ist.

11. Chirurgische Robotersystem (1) nach Anspruch 10, wobei die Basisverlängerung (522) ein teleskopisch bewegliches Gelenk (5221) umfasst, das ausgebildet ist, eine Länge der Basisverlängerung (522) zu ändern.

12. Chirurgisches Robotersystem (1) nach Anspruch 1, wobei der Aufhängungsarm (122, 722) und/oder der obere Einsatzteil (120, 420, 520, 620, 720) bogenförmig ist.

13. Chirurgisches Robotersystem (1) nach Anspruch 1, wobei der Roboterarm einen Schulterteil (130), einen mit dem Schulterteil (130) verbundenen Armträgerteil (131), einen mit dem Armträgerteil (131) verbundenen Oberarmteil (132), einen mit dem Oberarmteil (132) verbundenen Unterarmteil (133) und einen mit dem Unterarmteil (133) verbundenen Instrumententrägerteil (134) umfasst; wobei der Instrumententrägerteil (134) ausgebildet ist, mit dem chirurgischen Instrument (135) zu verbinden.

14. Chirurgisches Robotersystem (1) nach Anspruch 13, wobei der Armträgerteil (131) mit dem Schulterteil (130) schwenkbar verbunden ist, wobei der Oberarmteil (132) mit dem Armträgerteil (131) rotierbar verbunden ist, wobei der Unterarmteil (133) mit dem Oberarmteil (132) schwenkbar verbunden ist, und wobei der Instrumententrägerteil (134) mit dem Unterarmteil (133) schwenkbar verbunden ist.

15. Chirurgisches Robotersystem (1) nach Anspruch 1, wobei die Montagebasis (10, 20, 30) mit einem Rollenteil, einem festen Trägerteil und/oder einem Bewegungssteuerungsteil montiert ist.

## Revendications

1. Système robotique chirurgical (1), comprenant un module et un manipulateur (13), dans lequel le module comprend une base de montage (10, 20, 30), un composant de support (11, 21, 31) connecté à la base de montage (10, 20, 30) et une plate-forme d'orientation (12, 42, 52, 62, 72) reliée au composant de support (11, 21, 31) ; dans lequel le manipulateur (13) est connecté à la plateforme d'orientation (12, 42, 52, 62, 72) du module ; le manipulateur (13) comprend un bras robotique et un instrument chirurgical (135) connecté au bras robotique, et le manipulateur (13) est un manipulateur actif à centre de mouvement distant (13), dans lequel le bras robotique supportant l'instrument chirurgical l'instrument présente une configuration spatiale d'au moins quatre degrés de liberté, permettant à l'instrument chirurgical de se déplacer autour d'un certain point dans l'espace grâce à une commande par algorithme, la plateforme d'orientation (12, 42, 52, 62, 72) comprenant une partie plateau supérieure (120, 420, 520, 620, 720) et un bras de suspension (122), dans lequel au moins une partie de montage (121, 421, 621, 721) est répartie sur la partie supérieure du plateau (120, 420, 520, 620, 720) pour connecter directement ou indirectement le manipulateur (13), et **caractérisé en ce que** la partie supérieure du plateau (120, 420, 520, 620, 720) est fixée au bras de suspension (122, 722), et le bras de suspension (122, 722) est relié de manière rotative au composant de support (11, 21, 31).

2. Système robotique chirurgical (1) selon la revendication 1, dans lequel le composant de support (11, 21, 31) comprend une première partie de support (110, 210, 310) fixée à la base de montage (10, 20, 30) et une seconde partie de support (111, 211, 311) reliée à la première partie de support (110, 210, 310).

3. Système robotique chirurgical (1) selon la revendication 2, dans lequel la deuxième partie de support (111, 211, 311) est mobile le long d'une direction d'extension de la première partie de support (110, 210, 310) et/ou du deuxième support la partie (111, 211, 311) peut tourner par rapport à la première partie de support (110, 210, 310).

4. Système robotique chirurgical (1) selon la revendication 2, dans lequel la deuxième partie support (111, 211, 311) comprend une première branche support (1110, 2110, 3110) reliée à la première partie support (110, 210, 310), une deuxième branche de support (1111, 2111, 3111) connectée à la première branche de support (1110, 2110, 3110), et une troisième branche de support (1112, 2112, 3112) connectée à la deuxième branche de support (1111, 2111, 3111), dans lequel une direction d'extension de la première branche de support (1110, 2110, 3110) est perpendiculaire à une direction d'extension de la troisième branche de support (1112, 2112, 3112), de préférence,
la troisième branche de support (1112, 2112, 3112) est mobile le long d'une direction d'extension de la deuxième branche de support (1111, 2111, 3111), et/ou la première branche de support (1110, 2110, 3110) est mobile le long de la direction d'extension de la première pièce de support (110, 210, 310).

5. Système robotique chirurgical (1) selon la revendication 1, dans lequel la plate-forme d'orientation (12, 42, 52, 62, 72) peut tourner par rapport au composant de support (11, 21, 31).

6. Système robotique chirurgical (1) selon la revendication 1, dans lequel la partie supérieure du plateau (120, 420, 520, 620, 720) est en forme d'arc ou en forme de griffe.

7. Système robotique chirurgical (1) selon la revendication 6, dans lequel chaque partie de montage (121, 421, 621, 721) peut tourner par rapport à la partie supérieure du plateau (120, 420, 520, 620, 720).

8. Système robotique chirurgical (1) selon la revendication 6, dans lequel chaque partie de montage (121, 421, 621, 721) est mobile sur la partie supérieure du plateau (120, 420, 520, 620, 720).

9. Système robotique chirurgical (1) selon la revendication 1, dans lequel la partie plateau supérieur (120, 420, 520, 620, 720) comprend un support central (6200) et une pluralité d' éléments rotatifs (6201), une partie de montage (121, 421, 621, 721) est réparti sur chaque élément rotatif (6201), le support central est relié en rotation à un élément rotatif adjacent (6201), et chaque élément rotatif (6201) est relié en rotation à un élément rotatif adjacent (6201, 6201) à travers la pièce de montage correspondante (121, 421, 621, 721).

10. Système robotique chirurgical (1) selon la revendication 1, dans lequel la plate-forme d'orientation (12, 42, 52, 62, 72) comprend en outre une pluralité d'extensions de base (522), chaque extension de base (522) forme une première rotation connexion avec la partie plateau supérieure (120, 420, 520, 620, 720), chaque extension de base (522) forme une seconde connexion rotative avec le manipulateur (13), et un axe de la première connexion rotative est parallèle à un axe de la deuxième rotation connexion, de préférence,
un axe de la première liaison rotative est colinéaire avec un axe de la seconde liaison tournante.

11. Système robotique chirurgical (1) selon la revendication 10, dans lequel l'extension de base (522) comprend une articulation mobile télescopique (5221) configurée pour modifier une longueur de l'extension de base (522).

12. Système robotique chirurgical (1) selon la revendication 1, dans lequel le bras de suspension (122, 722) et/ou la partie plateau supérieur (120, 420, 520, 620, 720) est en forme d'arc.

13. Système robotique chirurgical (1) selon la revendication 1, dans lequel le bras robotique comprend une partie d'épaule (130), une partie de support de bras (131) reliée à la partie d'épaule (130), une partie supérieure de bras (132) reliée à la partie support de bras (131), une partie bras inférieur (133) reliée à la partie bras supérieur (132), et une partie support d'instrument (134) reliée à la partie bras inférieur (133) ; dans lequel la partie support d'instrument (134) est configurée pour se connecter à l'instrument chirurgical (135).

14. Système robotique chirurgical (1) selon la revendication 13, dans lequel la partie de support de bras (131) est reliée de manière pivotante à la partie d'épaule (130), la partie supérieure du bras (132) est reliée de manière rotative à la partie de support de bras (131), la partie bras inférieur (133) est reliée de manière pivotante à la partie bras supérieur (132), et la partie support d'instrument (134) est reliée de manière pivotante à la partie bras inférieur (133).

15. Système robotique chirurgical (1) selon la revendication 1, dans lequel la base de montage (10, 20, 30) est montée avec une partie roulette, une partie support fixe et/ou une partie de commande de mouvement.
